# EUROPEAN PATENT APPLICATION

(11) **EP 2 036 986 A1**
(43) Date of publication of application: **18.03.2009**
(21) Application number: 07744283.8
(22) Date of filing: 28.05.2007
(51) Int. Cl.: C12Q 1/02, C12N 5/10, C12N 15/09

(54) **CONTINUOUS ASSAY METHOD FOR MEASURING RECEPTOR- AND ION CHANNEL-MEDIATED CALCIUM RESPONSE AND SUBSEQUENT GENE EXPRESSION**

(30) Priority: 26.05.2006 JP 2006146987
(71) Applicant: Noacell Science Co., Ltd., Settsu-shi Osaka 566-0001 (JP)
(72) Inventor: ARAKI, Naohiro, Itano-gun, Tokushima 7711273 (JP); YABUSHITA, Hisatoshi, Tokushima-shi, Tokushima 7710144 (JP); SUZUKI, Yoshikazu, Naruto-shi, Tokushima 7720012 (JP); IIDA, Mitsuru, Naruto-shi, Tokushima 7720017 (JP)
(74) Representative: Dorn, Dietmar
(86) International application number: PCT/JP2007/060849
(87) International publication number: WO 2007/139080

(57) **Abstract**

A method of utilizing a calcium-responding photoprotein selected from the group consisting of the calcium-responding aequorin photoprotein from jellyfish, and its functional equivalents, and measuring rise in intracellular calcium; and continuously thereafter measuring, with the cells left alive within the same container, and employing the same substrate, induction and suppression of gene expression, utilizing a reporter-gene assay that uses and measures a reporter gene selected from the group consisting of calcium non-responding *Renilla* luciferase and its functional equivalents.

SEQUENCE LISTING

<110> MITSURU IIDA

<120> Continuous Assay Technique for Measuring Receptor- and
Ion-Channel-Mediated Calcium Response and Ensuing Gene Expression

<130> 060526P07

<160> 8

<170> PatentIn version 3.1

<210> 1
<211> 46
<212> DNA
<213> Human

<400> 1
ctggctgacgtcagtagagacatcccattgacgtcatacgtggtac @@ 46

<210> 2
<211> 46
<212> DNA
<213> Human

<400> 2
cacgtatgacgtcaatgggatgtctctactgacgtcagccaggtac 46

<210> 3
<211> 30
<212> DNA
<213> Human

<400> 3
gggaagcttatgctttatgatgttcctgat 30

<210> 4
<211> 30
<212> DNA
<213> Human

<400> 4
ggtaccttaggggacagctccaccgtagag 30

<210> 5
<211> 39
<212> DNA
<213> Human

<400> 5
tgatgtaagcttgaccttcatgatcccaagcttcccctc 39

<210> 6
<211> 39
<212> DNA
<213> Human

<400> 6
gaggggaagcttgggatcatgaaggtcaagcttacatca 39

<210> 7

<211>  38
<212>  DNA
<213>  Human

<400>  7
ctcgagtctagaccaccatggcccgctcgctgacctgg                                38


<210>  8
<211>  24
<212>  DNA
<213>  Human

<400>  8
ggtacctcacagcaggttgatctc                                              24

## Description

### Technical Field

The present invention relates to methods for continuously measuring intracellular calcium response and gene expression, to screen for receptor agonists and antagonists.

### Background Art

In general protein receptors-which are distributed in the cell membrane surface and within the cytoplasm or nucleus, and which uniquely recognize and bind various physiologically active substances from the cell exterior and transmit information on the physiologically active substances to the cell interior or DNA-are grossly classified, according to the location where they range, into both cell surface receptors and cytoplasmic receptors (also termed "intracellular receptors"). Cell membrane surface receptors bind with hydrophilic physiologically active substances (peptides, cytokines, catecholamines, growth factors, etc.), which cannot pass through the cell membrane, and via a swift structural transformation convert from an inactive form to an active form, transmitting information on the physiologically active substances to the cell interior. Owing to differences in their protein structures and information transmission mechanisms, cell surface receptors are grossly divided into three groups: tyrosine kinase-coupled receptors, ligand-gated ion channels, and G-protein coupled receptors.
Below, an explanation focusing on G-protein coupled receptors will made, but inasmuch as it is known that with tyrosine kinase-coupled receptors and ligand-gated ion channels also, the binding of chemical compounds to the receptors elevates intracellular calcium in the same way as with G-protein coupled receptors, continuous assaying in the same manner is possible, but in the former case, because a relatively long time period is required for the action/expression by the receptors, during that interval, incorporating multiple assays is even more possible.

### BRIEF SUMMARY OF THE INVENTION

G-protein coupled receptors are categorized into three broad classes according to the type of second messenger within the cell (Non-Patent Document 1):
(A) G_{q}: phospholipase-C mediated intracellular-calcium responding;
(B) Gₛ: adenylate-cyclase activating, cAMP-concentration elevating; and
(C) Gᵢ: adenylate-cyclase inhibiting, cAMP-concentration lowering. Depending on the type of these second messengers, assays that have been employed include: a technique (FLIPR) employing Fluo3, Fura2, or a similar fluorescent dye that reacts with calcium, (Non-Patent Documents 2 and 3); a method that by an alpha-screen, flash-plate, ELISA or similar test quantitates the intracellular cAMP or free IP₃ (Non-Patent Document 4); and a method that measures the incorporation of radiolabeled GTPγS into Gα subunits (Non-Patent Document 5).

Furthermore, as improvements, methods using chimeric proteins such as Gα_{*16*/*15*} or G_{z}-which are single types of Gα subunit appearing uniquely in erythrocytes-that react with these three classes of GPCRs to substitute for Gₛ and Gᵢ in calcium reactions of the Gq class have been developed. (Non-Patent Documents 6, 7 and 8). Difficulties have been reported, however, such as the fact that with a number of GPCRs, the switch in the calcium reactions is not possible, as well as signals being weak and sensitivity not being sufficiently assurable (Non-Patent Document 6).
Consequently, it has been necessary to combine a number of the aforementioned techniques and conduct tests separately, especially in cases such as when random screening for ligands for orphan receptors, whose ligands are unknown.

Non-Patent Document 1: Nambi, P., and Aiyar, N. (2003) Assay Drug Dev. Technol. 1, 305-10.
Non-Patent Document 2: Nakayama, T., Kato, Y., Hieshima, K., Nagakubo, D., Kunori, Y., Fujisawa, T., and Yoshie, O. (2004) J. Immunol. 173, 2078-83.
Non-Patent Document 3: Fujii, R., Hosoya, M., Fukusumi, S., Kawamata, Y, Habata, Y, Hinuma, S., Onda, H., Nishimura, O., and Fujino, M. (2000) J. Biol. Chem. 275, 21068-74.
Non-Patent Document 4: Offermanns, S., and Simon, M. I. (1995) J. Biol. Chem. 270, 15175-80.
Non-Patent Document 5: Ferrer, M., Kolodin, G. D., Zuck, P., Peltier, R., Berry, K., Mandala, S. M., Rosen, H., Ota, H., Ozaki, S., Inglese, J., and Strulovici, B. (2003) Assay Drug Dev. Technol. 1, 261-73.
Non-Patent Document 6: Stables, J., Green, A., Marshall, F., Fraser, N., Knight, E., Sautel, M., Milligan, G., Lee, M., and Rees, S. (1997) Anal. Biochem. 252, 115-26.
Non-Patent Document 7: New, D. C., and Wong, Y H. (2004) Assay Drug Dev. Technol. 2, 269-80.
Non-Patent Document 8: Milligan, G., Marshall, F., and Rees, S. (1996) Trends Pharmacol. Sci. 17, 235-7.
Non-Patent Document 9: Fitzgerald, L. R., Mannan, I. J., Dytko, G. M., Wu, H. L., and Nambi, P. (1999) Anal. Biochem. 275, 54-61.
Non-Patent Document 10: Bresnick, J. N., Skynner, H. A., Chapman, K. L., Jack, A. D., Zamiara, E., Negulescu, P., Beaumont, K., Patel, S., and McAllister, G. (2003) Assay Drug Dev. Technol. 1, 239-49.
Non-Patent Document 11: Kent, T. C., Thompson, K. S., and Naylor, L. H. (2005) J. Biomol. Screen. 10, 437-46.
Non-Patent Document 12: van Der Luit, A. H., Olivari, C., Haley, A., Knight, M. R., and Trewavas, A. J. (1999) Plant Physiol. 121, 705-14.

To date, it has been necessary to test for and measure, within independent assaying regimes, calcium response separately from induction and inhibition of gene expression. Examples measuring Gq signals by the calcium response of a flash-type photoprotein, and next measuring only the signals related to Gₛ. Utilizing the present invention, with cells left living, these multiple assays can be determined sequentially in the same cells, under the same conditions, in the same reaction vessel using the same substrate, which therefore enables a more precise evaluation and which can serve to curtail costs considerably. One example would be that in measuring the activity of G-protein coupled receptors, the coupled Gₛ, Gᵢ and G_{q} have to be measured and sorted afterwards. The present invention makes it possible to determine such measurements in the same cells, under the same conditions, and moreover, in the same container, in a series of operations with the cells left alive. In addition, within the series of operations, checking for whether active inhibitors influence due to cytotoxicity is also made possible.

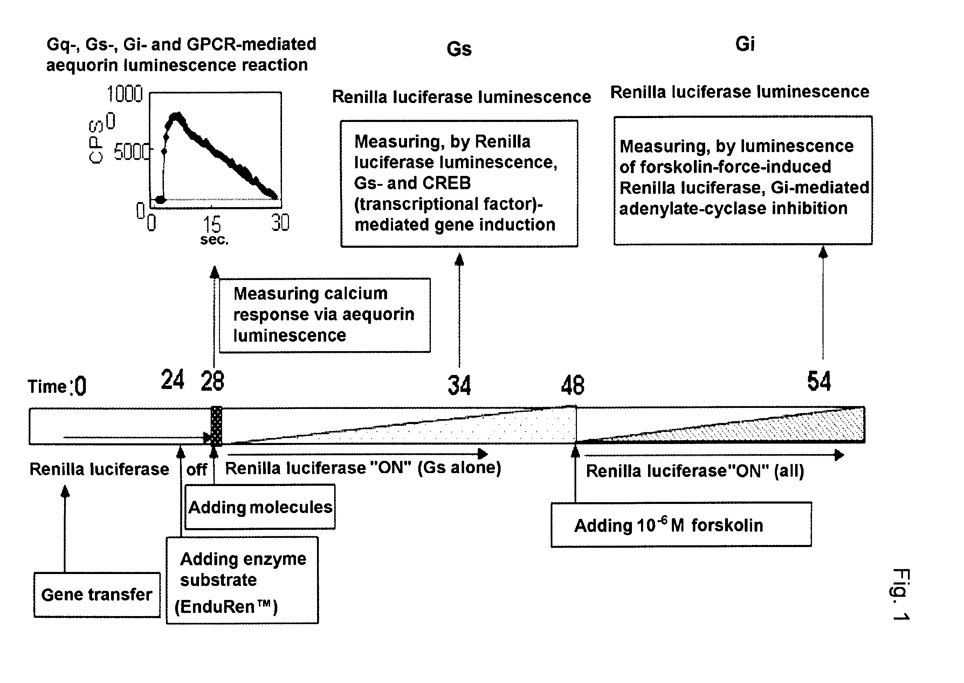

The present invention utilizes a calcium-responding, flash-type photoprotein (aequorin, obelin, etc.) localized in the cytoplasm or mitochondria, and a reporter gene, downstream of a promoter that responds to increase in intracellular cAMP, ligated to sea-pansy-derived, calcium-insensitive *Renilla* luciferase to measure G-protein-coupled-receptor-mediated elevation in intracellular calcium. Utilizing a coelenterazine inducer that can remain present within a cell stably for long time periods, to begin with the calcium-responding photoprotein is measured, and thereafter, several hours' culturing is continued and the activity of *Renilla* luciferase induced by an increase in cAMP is measured by a reporter assay. Furthermore, forskolin, which activates adenylate-cyclase, is continuously added to the cells, and whether the compound inhibits the activation of adenylate-cyclase is investigated by measuring the activity of *Renilla* luciferase. Finally, the cytotoxicity is evaluated by adding ligands for cytoplasmic GPCRs that appear in the cells and measuring the calcium response, and that the inhibition of adenylate-cyclase activation has not been caused by cytotoxicity is checked (Fig. 1). The aequorin reaction is triggered by calcium, and exhibits flash-type luminescence. In contrast, *Renilla* luciferase does not undergo influence from calcium, requires dissolved enzymes in reactions, and catalyzes long-term luminescence in the presence of a substrate at saturation density. That is, the method is one in which both the calcium-responding photoprotein (aequorin) and *Renilla* luciferase employ coelenterazine as a substrate, but with the reaction mechanisms differing, and with long-term, continuous-type coelenterazine inducer being used jointly, three different assays (calcium-responding, cAMP-elevating, and cAMP-increase blocking) are determined at once without adding fresh substrate.
Finally, the present invention is not limited to induction or suppression of cAMP (Non-Patent Documents 5, 10 and 11), but is utilizable in continuous measurements of the activation and suppression of other transcription, such as is mediated by multiple-response elements (MREs) as *cis*-elements employed upstream of *Renilla* luciferase (Non-Patent Document 9), and by calcium-responding transcription-factor response elements (AP-1, TPA, and serum-response elements [SREs]).

### Effects of the Invention

As has been explained in the foregoing, an advantage of the present invention is that by employing the same substrate intracellularly, with *Renilla*-derived luciferase as a reporter gene, to cause calcium-responding aequorin and the induction/suppression of the gene expression that thereupon occurs to express themselves together, the calcium response and genetic-expression response can be continuously assayed at once. Particularly when the invention is utilized with GPCRs as the assay subject, by causing Gα*₁₆*, calcium-responding aequorin, and as the reporter gene, *Renilla*-derived luciferase, which is induced by cAMP, to express themselves together intracellularly, an advantage is that all of the responses of the three known types of GPCR can be assayed at once. A further advantage is that by continuously observing the results of the first-time calcium response, and the cAMP-mediated induction of *Renilla*-derived luciferase and the inhibition of Renilla luciferase forcibly induced by forskolin, from which type of GPCR the reaction derives can be discriminated. Moreover, with wildtype coelenterazine, which has been employed to date as a substrate, being unstable within cells and disintegrating in a matter of minutes after its addition to a cell, continuous assaying has not been possible. With the present invention, utilizing a substrate that can remain present stably within cells has enabled the assaying at one time of calcium-responding aequorin proteins and of *Renilla*-derived luciferase reactions while the cell remains alive. Lastly, adding ligands for cytoplasmic GPCRs and measuring the calcium response has enabled a continuous assay within a series of operations whereby the cytotoxicity is evaluated, and whether the inhibition of adenylate-cyclase activation has arisen on account of the cytotoxicity is verified.

### Brief Description of Drawings

Fig. 1 is an outline diagram illustrating a measuring scheme for a continuous assay overall of the present invention.
Fig. 2 is a map of a plasmid utilizing the present invention.
Fig. 3 is a graph plotting results in Embodiment 1 wherein the calcium response of G_{q}-coupled GPCRs was measured with aequorin.
Fig. 4 is a graph plotting results in Embodiment 1 wherein cAMP-mediated genetic induction by G_{q}-coupled GPCRs was measured with *Renilla* luciferase. Following sample addition, the measurement in Fig. 3 was continued and after five hours the *Renilla*-luciferase mediated luminescence was measured.
Fig. 5 is a graph plotting results in Embodiment 2 wherein the calcium response of Gₛ-coupled GPCRs was measured with aequorin.
Fig. 6 is a graph plotting results in Embodiment 2 wherein cAMP-mediated genetic induction by Gₛ-coupled GPCRs was measured with *Renilla* luciferase. Following sample addition, the measurement in Fig. 5 was continued and after five hours the *Renilla*-luciferase mediated luminescence was measured.
Fig. 7 is a graph plotting results in Embodiment 3 wherein the calcium response of Gᵢ-coupled GPCRs was measured with aequorin.
Fig. 8 is a graph plotting results in Embodiment 3 wherein the Gᵢ-coupled GPCR suppression of cAMP synthesis was measured with *Renilla* luciferase. Following sample addition, the measurement in Fig. 7 was continued, and after 24-hours' culturing, 10⁻⁶ M of forskolin was added, and after further cultivating for five hours, the *Renilla*-luciferase was measured.
Fig. 9 diagrams measurement results in Embodiment 4, comparing wildtype coelenterazine and EnduRen^{™} as luminescent matrices.
Fig. 10 is a graph plotting results in Embodiment 4 wherein wildtype coelenterazine and EnduRen^{™} were employed as luminescent matrices and the forskolin-stimulated synthesis of cAMP was continuously measured with *Renilla* luciferase. Following sample addition, the measurement in Fig. 9 was continued and after five hours the *Renilla*-luciferase mediated luminescence was measured.

### Best Mode for Carrying Out the Invention

For the stable coelenterazine inducer set forth by the present invention, Promega Corp.'s EnduRen^{™} is employed, but as long as the inducer is stably viable for at least no less than 24 hours, it is not thereby limited. Likewise, neither are the plasmids limited to those set forth in the present-invention embodiments; of course any that provide the same functioning are acceptable.
It will be appreciated that in the following embodiments, while cells co-expressing calcium-responding aequorin and *Renilla* luciferase are added to the molecules (ligands), as a matter of course, the molecules may be added to such co-expressing cells.
Below, the present invention will be described further according to embodiments thereof, yet the present invention is not limited to these embodiments.

### Details of Protocol for Constructing pGL3 CRE Renilla Luciferase Plasmids

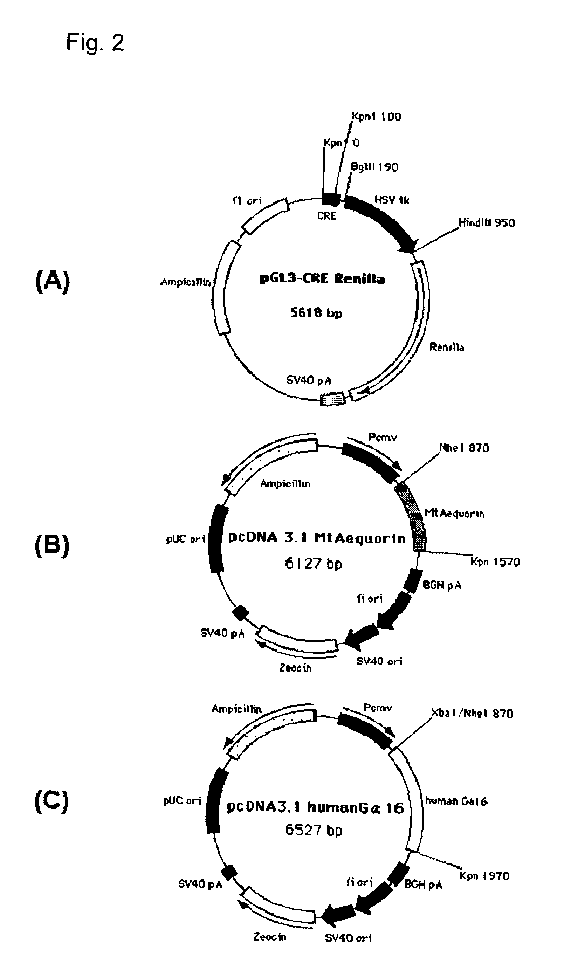

- The thymidine-kinase promoter segment, the (-41 to +30) segment, was excised from pRL-TK (Promega Corp.), and inserted into BglII-HindIII from the pGL3 basic vector (Promega Corp.) to create pGL3-tk.
- cAMP response element (CRE) oligonucleotides having KpnI ends at 5' and 3'-
   (1) 5'-ctggcTGACGTCAgtagagacatcccatTGACGTCAtacgtggtac-3',
   (2) 5'-cacgtaTGACGTCAatgggatgtctctacTGACGTCAgccaggtac-3'
      (the underlining indicating the CREs)-
      were bound with DNA-ligase, and the oligonucleotides that had become triple-stranded were introduced into the KpnI sites of the pGL3-tk to construct pGL3-CRE *luc*+ into which a total of six CREs had entered.
- The fluorescent luciferase gene (*luc*+) was excised from pGL3-CRE *luc*+ with the restriction enzymes Ncol and Xbal. Next, the sea-pansy luciferase *(Renilla)* gene was excised from pRL-TK with Ncol and Xbal, and the *Renilla* gene was introduced into the *luc*+ gene-excised pGL3-CRE to construct pGL3-CRE *Renilla* (Fig. 2A).

### Details of Protocol for Constructing Mitochondria-Localized Aequorin Expression Vector (pcDNA MtAEQ)

- Utilizing
   aeq-s1: 5'-GGGAAGCTTATGCTTTATGATGTTCCTGAT-3', and
   aeq-a1: 5'-GGTACCTTAGGGGACAGCTCCACCGTAGAG-3'
   as amplification primers, the aequorin-coding region (576 bp) was amplified by the PCR method with pBIN19NucAeq plasmid (Non-Patent Document 12) for the template.
- A mitochondrial transit signal sequence (100 bp) in exon 1 of the mitochondrial cytochrome c oxidase subunit 8A gene was amplified by the PCR method using the following primers, with human genomic DNA from a healthy individual as a template:
   (cyt-aeq-fus-p2:
   5'-TGATGTAAGCTTGACCTTCATGATCCCAAGCTTCCCCTC-3', and
   cyt-aeq-fus-p3:
   5'-GAGGGGAAGCTTGGGATCATGAAGGTCAAGCTTACATCA-3').
- The two obtained amplification products (576 bp and 100 bp) were annealed by recombinant PCR and the obtained product (676 bp) was treated with the restriction enzymes KpnI and Nhel, and cloned with Kpnl- and Nhel-treated pcDNA 3.1 (Invitrogen Corp.) to construct the aequorin expression vector pcDNA 3.1 MtAequorin (pcDNA MtAEQ) (Fig. 2B).

### Details of Protocol for Constructing pcDNA Gα16 Plasmids

- The gene for the human Gα₁₆ subunit was amplified from human leukocyte poly-A RNA by the RT-PCR utilizing the following primers:
   Gα₁₆-s2:
   5'-CTCGAGTCTAGACCACCATGGCCCGCTCGCTGACCTGG-3',
   Gα₁₆-a2: 5'-GGTACCTCACAGCAGGTTGATCTC-3').
- The amplification product was subcloned with a cloning vector (pGEM-T: Promega Corp.) to determine the base sequence, and was confirmed to be identical with that recorded in GenBank as the human Gα₁₆ subunit gene.
- The aforesaid DNA was cloned on the Nhel and Kpn1 sites in the expression vector pcDNA3.1 (Invitrogen Corp.) having CMV promoters as DNA inserts, to construct the Gα₁₆ subunit expression vector pcDNA 3.1 human Gα₁₆ (pcDNAGα₁₆) (Fig. 2C).

### Embodiment 1

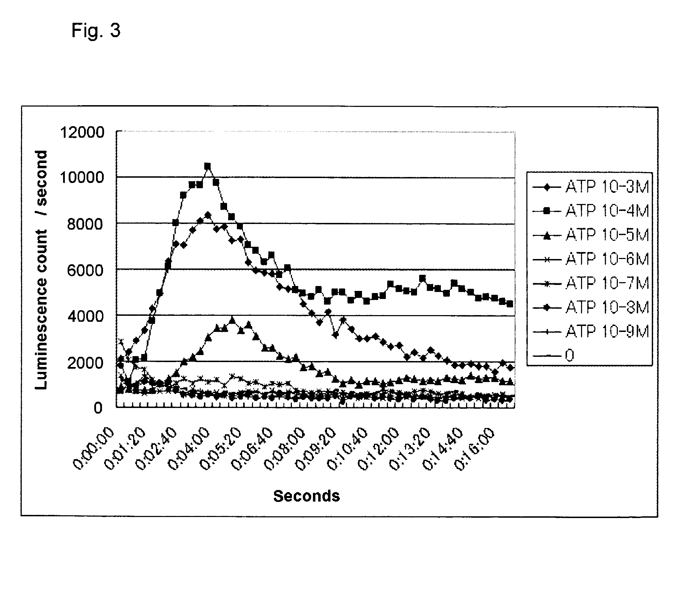

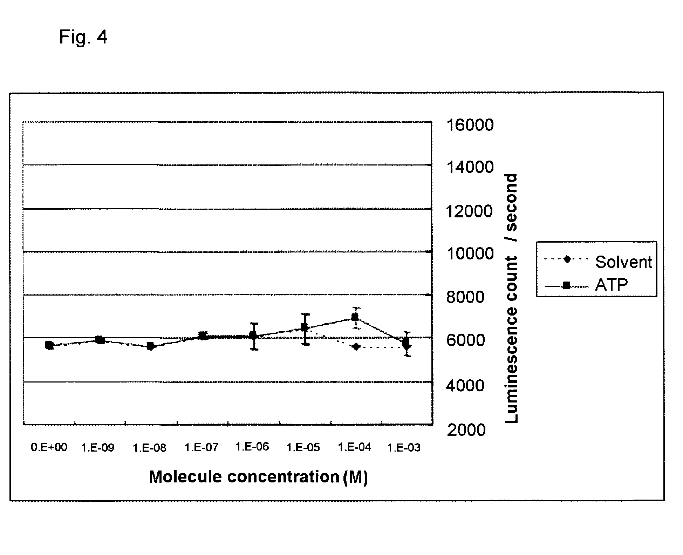

In the present embodiment, the response of Gq-coupled GPCRs was continuously measured.
To begin with, plasmids (pcDNA MtAEQ, pcDNA Gα₁₆, and pGL3 CRE-Renilla) were introduced into CHO cells. Following an overnight culturing, EnduRen^{™} was added as a substrate, the combination was cultivated four hours, and from 10⁻³ to 10⁻⁹ M sample (ATP) was added. For 30 seconds directly post-addition, the aequorin-mediated luminescence was continuously measured. The results are graphed in Figs. 3 and 4.
In detail, a CHO cell line (ATCC-deposited cells) that expresses purine receptors was seeded into 10 cm² Petri dishes at a 1.5 x 10⁵ cells/ml cellular concentration.
The next day, 2.2 µg Gα₁₆ (pcDNA Gα₁₆), 3 µg aequorin-expressing plasmids (pcDNA MtAequorin), and 3 µg cAMP-responding *Renilla* luciferase plasmids (pGL3 CRE-*Renilla*) were transfected into each Petri dish employing 18 µL of FuGENE® 6 (by Roche Diagnostics). The following day, 400 µL of trypsin/EDTA solution was added to the Petri dishes, and the cells were dissociated and suspended in 13 mL of DMEM/F12 FCS nutrient broth. As a stabilizing substrate, 6.5 µL of 60 mM EnduRen^{™} was added to the suspension solution, and after four hours' culturing, 90 µL of the cell suspension solution was added into each well of a 96-well plate into which 10 µL ATP (adenosine 5'-triphosphate disodium hydrate) as purine-receptor ligands had been added at 10⁻³ to 10⁻⁹ M concentration. Results of measuring the change in luminescent intensity for 30 seconds immediately following the suspension addition, employing the ARVO^{™} luminometer by Berthold Technologies GmbH, were diagrammed (Fig. 3). In Fig. 3, the vertical axis indicates the per-second luminescent intensity (count per second), and the horizontal axis, the measuring time (seconds). Thereafter culturing was continued, and the results six hours later of measuring the activity of *Renilla* luciferase with the ARVO^{™} were diagrammed (Fig. 4). In Fig. 4, the vertical axis indicates the per-second luminescent intensity (count per second), and the horizontal axis, the ATP concentration. It will be understood that, with no induction of cAMP-mediated *Renilla* luciferase occurring, the purine receptors were G_{q}-coupled GPCRs.

### Embodiment 2

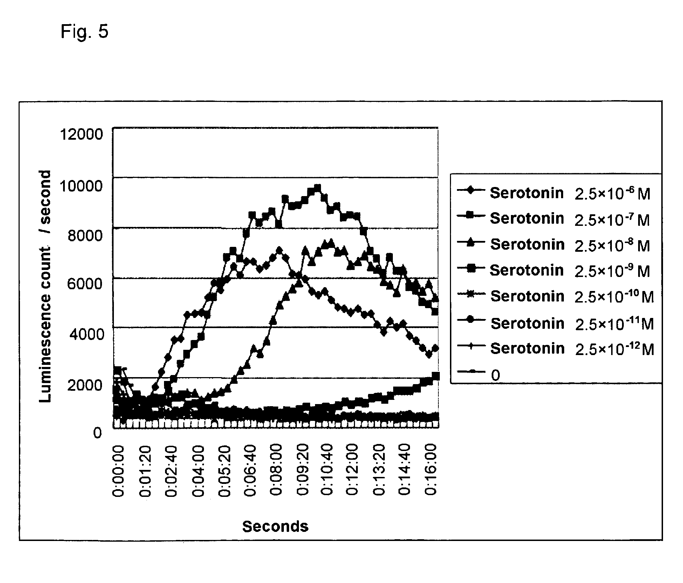

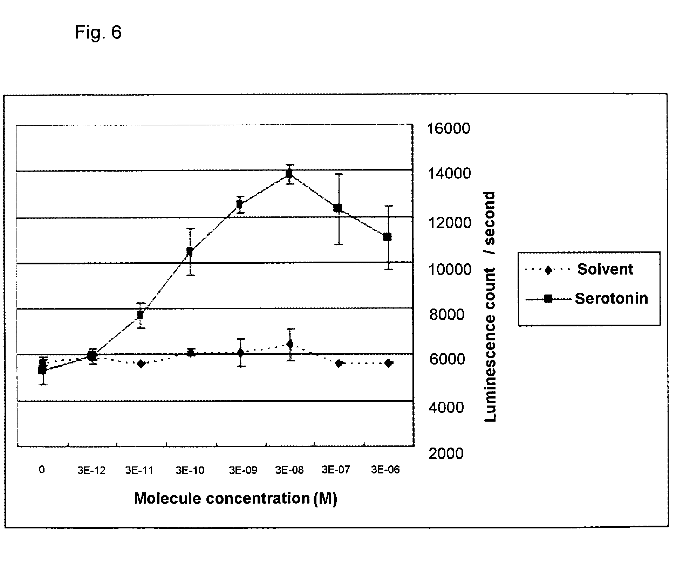

In the present embodiment, the response of Gₛ-coupled GPCRs was continuously measured.
To begin with, plasmids (pcDNA MtAEQ, pcDNA Gα₁₆, and pGL3 CRE-Renilla) were introduced into serotonin-receptor (5HT2A) expressing cells. Following an overnight culturing, EnduRen^{™} was added as a substrate, the combination was cultivated four hours, and from 2.5 × 10⁻⁶ to 2.5 × 10⁻¹² M sample (serotonin) was added. For 30 seconds directly post-addition, the aequorin-mediated luminescence was continuously measured. The results are graphed in Figs. 5 and 6.
In detail, a serotonin receptor (5HT2A) was transfected into a CHO cell line (ATCC-deposited cells), and, in the same manner as in Embodiment 1, Gα₁₆, aequorin-expressing plasmids, and cAMP-responding *Renilla* luciferase plasmids were transfected into the stably expressing cell line and the cell suspension solution was adjusted.
As a stable substrate, 30 µm EnduRen^{™} was added, and after four hours' culturing, 90 µL of the cell suspension solution was added into each well of a 96-well plate into which 10 µL serotonin hydrochloride as serotonin-receptor ligands had been added at 2.5 × 10⁻⁶ to 2.5 × 10⁻¹² M concentration. Results of measuring the change in luminescent intensity for 30 seconds immediately following the suspension addition, employing the ARVO^{™} luminometer by Berthold Technologies GmbH, were diagrammed (Fig. 5). In Fig. 5, the vertical axis indicates the per-second luminescent intensity (count per second), and the horizontal axis, the measuring time (seconds). Thereafter culturing was continued for six hours, and the results of measuring the activity of *Renilla* luciferase were diagrammed (Fig. 6). In Fig. 6, the vertical axis indicates the per-second luminescent intensity (count per second), and the horizontal axis, the serotonin concentration. From the fact that cAMP-mediated increase in (induction of) *Renilla* luciferase was confirmed, it will be understood that the serotonin receptors were Gs-coupled GPCRs.

### Embodiment 3

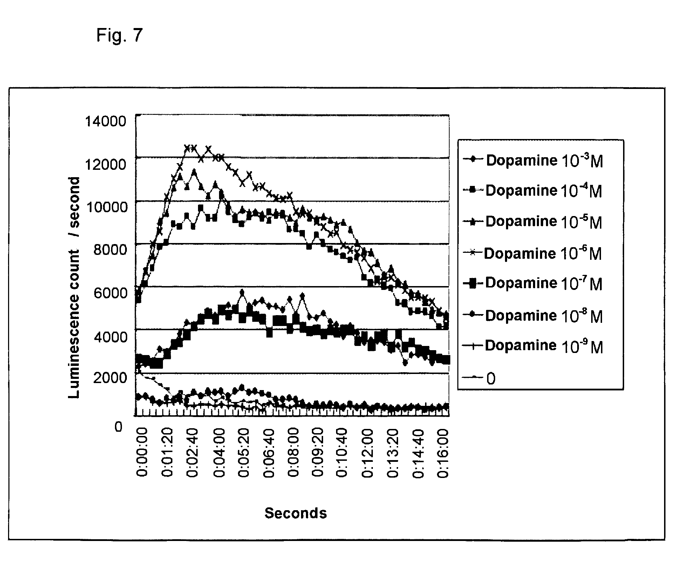

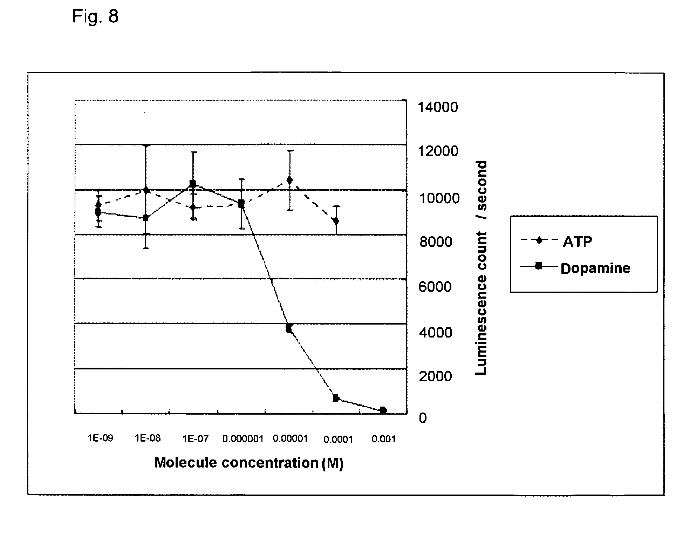

In the present embodiment, the response of Gᵢ-coupled GPCRs was continuously measured.
To begin with, plasmids were introduced into dopamine-receptor-(D2RS) expressing cells. Following an overnight culturing, EnduRen^{™} was added as a substrate, the combination was cultivated four hours, and from 10⁻³ to 10⁻⁹ M sample (dopamine) was added. For 30 seconds directly post-addition, the aequorin-mediated luminescence was continuously measured. The results are graphed in Figs. 7 and 8.
In detail, dopamine receptors (D2RS) were transfected into a CHO cell line (ATCC-deposited cells), and, in the same manner as in Embodiment 1, Gα₁₆, aequorin-expressing plasmids, and cAMP-responding *Renilla* luciferase plasmids were transfected into the stably expressing cell line and the cell suspension solution was adjusted.
As a stable substrate, 30 µm EnduRen^{™} was added, and after four hours' culturing, 90 µL of the cell suspension solution was added into each well of a 96-well plate into which 10 µL dopamine hydrochloride as dopamine-receptor ligands had been added at 1.0 × 10⁻³ to 1.0 × 10⁻⁹ M concentration. Results of measuring the change in luminescent intensity for 30 seconds immediately following the suspension addition, employing the ARVO^{™} luminometer by Berthold Technologies GmbH, were diagrammed (Fig. 7). In Fig. 7, the vertical axis indicates the per-second luminescent intensity (count per second), and the horizontal axis, the measuring time (seconds). The following day, 10 µL of 10⁻⁵ M forskolin was added, and culturing was continued for six hours. The results of measuring the activity of *Renilla* luciferase were diagrammed (Fig. 8). In Fig. 8, the vertical axis indicates the per-second luminescent intensity (count per second), and the horizontal axis, the dopamine concentration. The forskolin-stimulated activity of *Renilla* luciferase declined. From these results it will be understood that the dopamine receptors were Gᵢ-coupled GPCRs.

### Embodiment 4

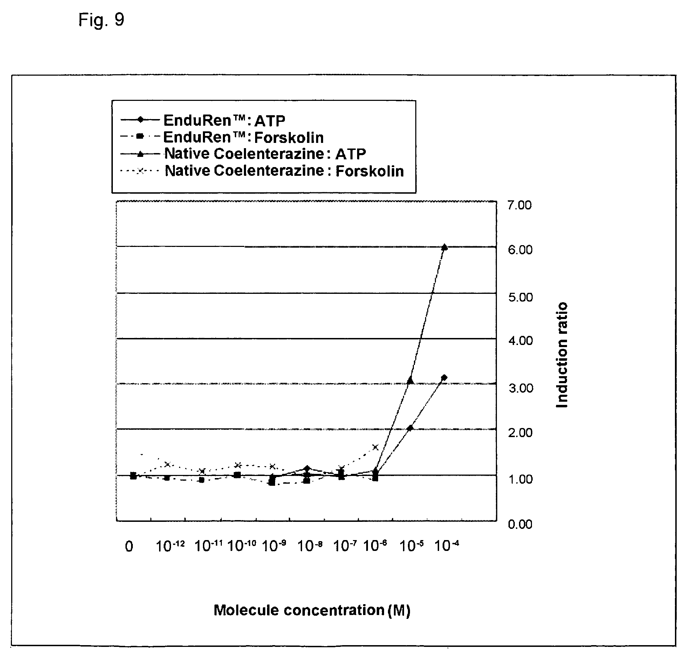

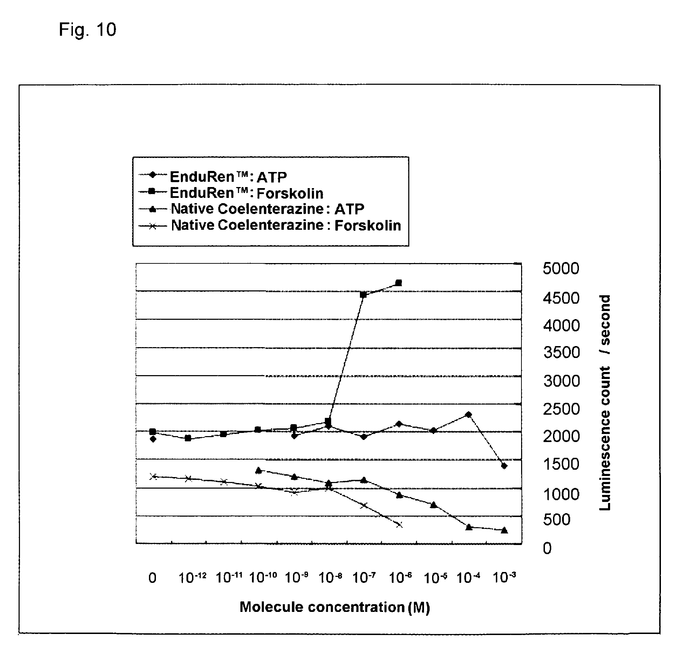

Wildtype coelenterazine and, as a stable substrate, EnduRen^{™} ere employed as luminescent substrates for enzymes, and the continuous-measurement regimes were compared. Plasmids were introduced into CHO cells. Following an overnight culturing, either 30 µm EnduRen^{™} or 30 µm wildtype coelenterazine was added as a substrate, the combinations were cultivated four hours, and sample (ATP at from 10⁻³ to 10⁻⁹ M; forskolin at from 10⁻⁶ to 10⁻¹² M) was added. For 30 seconds directly post-addition, the aequorin-mediated luminescence was continuously measured. The relative intensity divided by the solvent volume was indicated as the induction magnification. The results are diagrammed in Fig. 9.
In detail, in the same manner as in Embodiment 1, Gα₁₆, aequorin-expressing plasmids, and cAMP-responding *Renilla* luciferase plasmids were transfected into a CHO cell line (ATCC-deposited cells), and the cell suspension solution was adjusted. As a stabilizing substrate, 6.5 µL of 30 µm EnduRen^{™} and, likewise, 30 µm wildtype coelenterazine were added, and after four hours' culturing, 90 µL of the cell suspension solution was added into each well of a 96-well plate into which 10 µL ATP (adenosine 5'-triphosphate disodium hydrate) as purine-receptor ligands had been added at 10⁻³ to 10⁻⁹ M concentration. The relative activity by the luminescent-intensity maximum value measured for 30 seconds immediately following the suspension addition, employing the ARVO^{™} luminometer by Berthold Technologies GmbH, was diagrammed (Fig. 9). In Fig. 9, the vertical axis indicates the relative activity (fold induction) divided by the solvent volume, and the horizontal axis, the ligand concentration (M). ATP and forskolin were employed as the ligands. It will be understood that with either substrate, the ATP-stimulated calcium response can be measured by the aequorin luminescence. For comparison, culturing was thereafter continued, and the results six hours later of measuring the activity of *Renilla* luciferase with the ARVO^{™} were diagrammed (Fig. 10). In Fig. 10, the vertical axis indicates the per-second luminescent intensity (count per second), and the horizontal axis, the ligand concentration.
It will be understood that with EnduRen^{™}, cAMP induction brought about by forskolin could be detected, whereas with wildtype coelenterazine detection was not possible.

### Industrial Applicability

The present invention relates to methods of continuously measuring intracellular calcium response and gene expression, to screen for receptor agonists and antagonists; and inasmuch as the invention enables a multiple assays to be carried out continuously, it naturally has industrial utility.

## Claims

1. A method of:
utilizing a calcium-responding photoprotein selected from the group consisting of the calcium-responding aequorin photoprotein from jellyfish, and its functional equivalents, and measuring rise in intracellular calcium; and
continuously thereafter measuring, with the cells left alive within the same container, and employing the same substrate, induction and suppression of gene expression, utilizing a reporter-gene assay that uses and measures a reporter gene selected from the group consisting of calcium non-responding *Renilla* luciferase and its functional equivalents.

2. A procedure of, with cells left alive within the same container, and employing the same substrate, carrying out continuously, or selecting according to need and carrying out continually:
a technique of utilizing G-protein-coupled receptors, Gα₁₆ subunits or chimeric proteins of other α subunits, and cells that express calcium-responding photoproteins, and measuring, using the calcium-responding photoproteins, rise in intracellular calcium occurring by the binding-on of molecules;
a reporter-gene assay that measures induction and suppression of gene expression, as a reporter gene selected from the group consisting of calcium non-responding *Renilla* luciferase and its functional equivalents; and
a technique of evaluating cytotoxicity by adding ligands for cytoplasmic GPCRs that manifest in cells and measuring the calcium response.

3. A procedure of, with cells left alive within the same container, and employing the same substrate, carrying out continuously at once, or selecting according to need and carrying out continually:
a technique of utilizing tyrosine kinase-coupled receptors, and cells that manifest calcium-responding photoproteins, and measuring, using the calcium-responding photoproteins, rise in intracellular calcium occurring by the binding-on of molecules; and
a reporter-gene assay that measures induction and suppression of gene expression, as a reporter gene selected from the group consisting of calcium non-responding *Renilla* luciferase and its functional equivalents.

4. A procedure of, with cells left alive within the same container, and employing the same substrate, carrying out continuously at once, or selecting according to need and carrying out continually:
a technique of utilizing ion-channels, and cells that manifest calcium-responding photoproteins, and measuring, using the calcium-responding photoproteins, rise in intracellular calcium occurring by the binding-on of molecules; and
a reporter-gene assay that measures induction and suppression of gene expression, as a reporter gene selected from the group consisting of calcium non-responding *Renilla* luciferase and its functional equivalents.

5. A method as set forth in any of claims 1 through 4, wherein ligands selected from agonists acting on the receptors, and antagonist-acting substances are detected.

6. A cell line expressing both the jellyfish-derived calcium-responding aequorin photoprotein and its functional equivalents, and a calcium non-responding reporter gene.

7. A calcium-response- and gene-expression-assaying kit including:
plasmid DNA expressing the jellyfish-derived calcium-responding aequorin photoprotein, and its functional equivalents; and
a reporter-gene plasmid selected from the group consisting of and calcium non-responding *Renilla* luciferase and its functional equivalents.
